(19) Europäisches Patentamt
European Patent Office
Office européen des brevets

(11) Veröffentlichungsnummer: **0 151 931**
**B1**

(12) # EUROPÄISCHE PATENTSCHRIFT

(45) Veröffentlichungstag der Patentschrift: **07.06.89**

(51) Int. Cl.[4]: **C 07 F 7/00**, B 01 J 31/38, B 01 J 31/22, B 01 J 21/06, C 08 K 5/00, C 09 D 7/12

(21) Anmeldenummer: **85100200.6**

(22) Anmeldetag: **10.01.85**

(54) **Wasserlösliche Zirkonsäureester.**

(30) Priorität: **11.02.84 DE 3404949**

(43) Veröffentlichungstag der Anmeldung: **21.08.85 Patentblatt 85/34**

(45) Bekanntmachung des Hinweises auf die Patenterteilung: **07.06.89 Patentblatt 89/23**

(84) Benannte Vertragsstaaten: **BE DE FR GB IT NL SE**

(56) Entgegenhaltungen:
**EP-A- 0 092 756**
**DE-A- 2 204 531**
**DE-C- 1 122 049**

(73) Patentinhaber: **HÜLS AKTIENGESELLSCHAFT**
**Paul-Baumann-Strasse 1**
**D-4370 Marl 1 (DE)**

(72) Erfinder: **Barfurth, Dieter**
**Adenauerstrasse 34**
**D-5210 Troisdorf-Spich (DE)**
Erfinder: **Nestler, Heinz, Dr.**
**Rembrandtstrasse 73**
**D-5210 Troisdorf-Eschmar (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung sind Zirkonsäureester, die in Wasser ohne Zersetzung löslich und beständig sind.

Organische Derivate der Zirkonsäure $Zr(OH)_4$ sind vielseitig einsetzbare, reaktive Chemikalien. Besonders wichtige Vertreter dieser Gruppe sind Tetraalkylzirkonate wie z. B. Tetrapropylzirkonat oder Tetrabutylzirkonat und Zirkonsäurechelate, wobei die am häufigsten verwendeten Chelatbildner Acetylaceton und Triethanolamin sind. Beim Einsatz dieser Chelatbildner erhält man jedoch Chelate, die in nahezu allen üblichen Lösungsmitteln nur sehr schwer löslich sind, bzw. zu alkalisch reagierenden Verbindungen führen.

Diese bekannten Zirkonsäureester und -chelate werden u. a. eingesetzt als Katalysatoren für Polymerisations-, Polyadditions- und Ver- und Umesterungsreaktionen, als Vernetzer für polymere Substanzen wie Organopolysiloxane, Nitrocellulose oder hydroxylgruppenhaltige Harze, bei der Herstellung von Überzugsmitteln, Präzisionsgießformen, Textilveredlungsmitteln oder Hydrophobierungsmitteln und als Wirkstoff für die Glasoberflächenbehandlung. In jedem Fall geschieht dann die Anwendung im wasserfreien Medium bzw. wasserfreien Lösungsmitteln, obwohl aus wirtschaftlichen und ökologischen Gründen die Verwendung von Wasser vielfach wünschenswert ist. Die vielseitige Anwendbarkeit der Zirkonate als Katalysator und Vernetzer beruht auf der großen Reaktivität dieser Verbindungen. Gleichzeitig stört diese Reaktivität die Anwendung dieser Verbindungen immer dann, wenn Wasser im Reaktionsgemisch anwesend ist: Alkylzirkonate reagieren schon mit der Luftfeuchtigkeit, so daß Verdünnungen mit 96 %igem Alkohol bereits zu Ausfällungen zu Zirkonoxidhydrat führen.

Es ist deshalb bereits versucht worden, diese bekannten Zirkonverbindungen gegenüber der Einwirkung von Wasser zu stabilisieren. So wird z. B. in der DE-PS 22 04 531 vorgeschlagen, alkoholischen Lösungen von Zirkonpropylat oder -butylat Chelatbildner wie Acetylaceton oder Acetessigester, Hydroxycarbonsäuren oder mehrwertige Alkohole unterzumischen, wobei der Abgespaltene Alkohol neben dem ursprünglich im Zirkonat zu 20 bis 30 % ohnehin vorhandenen Lösungsalkohol verbleibt. Die dabei erhaltenen Produkte sind jedoch keine reinen Verbindungen, sondern Mischungen der Ausgangsprodukte mit Zirkonacetylacetonaten bzw. Zirkonchelaten der anderen Chelatbildner in alkoholischer Lösung. Wenn man beispielsweise ein auf diese Weise mit Acetylaceton erhaltenes Gemisch einengt, dann fällt aus dieser Lösung schwer lösliches Zirkonacetylacetonat als Feststoff aus.

Gemäß einer zweiten, in der DE-PS 11 22 049 beschriebenen Verfahrensweise werden wasserlösliche Metallalkoholate dadurch erhalten, daß Metallalkoholate niederer Alkohole mit speziellen längerkettigen Alkoholen umgesetzt werden. Diese Alkohole werden erhalten durch Anlagerung von Ethylenoxid an organische Verbindungen mit mindestens 4 C-Atomen und einem aktiven Wasserstoffatom. Im Falle des Zirkons entstehen dabei Verbindungen, die zwar erst nach mehrstündigem Stehen in Wasser hydrolysieren, deren $ZrO_2$-Gehalt allerdings stark reduziert ist; im Beispiel des dort genannten Zirkontetraethylats fällt er von 45,4 % auf 12,6 % ab.

Es bestand deshalb die Aufgabe, Zirkonsäureester mit hohem $ZrO_2$-Gehalt zu finden, die flüssig sind, die in Wasser ohne Ausfällung unlöslicher Hydrolysate löslich sind und deren wässrige Lösungen neutrale Reaktion zeigen.

In Erfüllung dieser Aufgabe wurden nun neue Zirkonsäureester der Formel

$$Zr(OR)_n(OR')_o\ C_5H_7O_2)_p$$

gefunden, in der

R = Alkylreste mit 1 bis 8 C-Atomen,

$R' = -[CH_2-CHR''-O]_q-R$,

R'' = H oder Methylgruppe bedeuten, der Rest $C_5H_7O_2$ für den Acetylacetonylrest steht

und

n = 0-2,

o = 1-3,

p = 1-2 sein kann mit der Bedingung, daß n + o + p = 4 ist

und

q = Werte von 1-8 annehmen kann.

Diese neuen, teilchelatisierten Zirkonsäureester sind bei Raumtemperatur flüssige Verbindungen, die sämtliche oben aufgeführten Nachteile nicht besitzen: Zur Herstellung von Lösungen benötigen sie keine Hilfslösemittel, sie sind mit Wasser in jedem Verhältnis mischbar, ohne zu hydrolysieren. Weiterhin sind sie im wünschenswerten Maße reaktiv und weisen einen relativ hohen $ZrO_2$-Gehalt auf.

Die Herstellung der erfindungsgemäßen Zirkonate kann auf an sich bekannte Weise durch Ver- oder Umesterung an sich bekannter Zirkonverbindungen erfolgen. Zweckmäßigerweise geht man von Tetraalkylzirkonaten aus, die der besseren Handhabbarkeit wegen in einem Überschuß des der Alkoxygruppe entsprechenden Alkohols gelöst im Handel erhältlich sind. Im allgemeinen werden deshalb ca. 70 bis 85 %ige alkoholische Lösungen der Tetraalkylzirkonate als Ausgangsprodukte eingesetzt.

Bei der praktischen Durchführung dieser bevorzugten Arbeitsweise wird das Zirkonat in einem

geeigneten Reaktionsgefäß mit 1 bis 3 Mol Glykolethern oder Glykoletherestern und mit bis zu 2 Mol Acetylaceton in der Wärme umgesetzt, wobei die Reaktionswärme oder kurzes Erwärmen auf eine Temperatur, die geringfügig unterhalb des Siedepunktes des vorhandenen Alkohols liegt, für die Durchführung der Umsetzung genügt. Anschließend wird sowohl der ursprünglich vorhandene als auch der durch Reaktion entstandene Alkohol — pro Mol Acetylaceton und Glykolether ein Mol — abdestilliert, zweckmäßigerweise im Vacuum bei mittlerer Temperatur.

Will man die Reaktion in zwei Stufen ablaufen lassen, so empfiehlt es sich, zunächst die Umsetzung mit dem Glykolether durchzuführen, den bis daher im Reaktionsgemisch vorhandenen Alkohol abzuziehen und anschließend mit Acetylaceton umzusetzen und nochmals einzuengen. Im Falle, daß man das Alkylzirkonat zuerst mit Acetylaceton reagieren läßt, besteht während des Einengens die Gefahr der zwischenzeitlichen Ausfällung fester Acetylacetonate, die dann unter Umständen nur schwer mit dem Glykolether weiterreagieren.

Unter den Begriff Glykolether, die zur Herstellung der neuen Verbindungen eingesetzt werden können, fallen sowohl Mono- als auch Polyglykolether sowie auch (Poly-)Glykoletherester, die der allgemeinen Formel

$$RO(CH_2—CHR''—O)_q—X$$

entsprechen, in der R für Alkylreste mit 1 bis 8 C-Atomen, R'' für Wasserstoff oder eine Methylgruppe, q für Werte von 1 bis 8, vorzugsweise von 1 bis 4 steht und X = H oder OC—P sein kann. Beispiele für entsprechende Glykolether (X = H) sind Glykolmonomethylether, Glykolmonoethylether, Glykolmonobutylether, Diglykolmonomethylether, Diglykolmonoethylether und Diglykolmonobutylether. Die Glykoletherester (X—OCR) umfassen beispielsweise Glykolmonomethyletheracetat oder Diglykolmonobutyletheracetat. Allgemein werden diese Glykoletherester auch als Alkylglykolcarboxylate bezeichnet. Beim Einsatz dieser Glykoletherester in die Synthese reagiert deren Estergruppierung mit einer Zr-OR-Gruppe unter Umesterung zu einem Carbonsäureester und Bildung der gewünschten Glykolethergruppierung der beanspruchten Verbindungen.

Es ist weiterhin möglich, Gemische unterschiedlicher Glykolether/Glykoletherester in die Reaktion einzubringen und das nach Abdestillation des Alkohols erhaltene Produkt dann direkt einzusetzen, ohne es in seine Einzelkomponenten aufzutrennen.

Die erfindungsgemäßen Zirkonchelate sind gelbe bis orange, mehr oder weniger viskose Flüssigkeiten. Sie sind ohne Hydrolyse in Wasser löslich. Sie sind überall dort einsetzbar, wo Zirkonate als Katalysatoren, Vernetzer etc. wirksam sind. Ihren besonderen Einsatz finden diese Verbindungen allerdings dort, wo andere Zirkonate aufgrund der Anwesenheit von Wasser oder Feuchtigkeit versagen oder auch nur Schwierigkeiten bereiten: Als Additiv in Wasserlacksystemen, als Vernetzer von Polyhydroxyverbindungen in Wasser, als Veresterungskatalysator oder in ähnlichen Systemen.

Die nachfolgenden Beispiele sollen Herstellung und Anwendungsmöglichkeiten der erfindungsgemäßen Zirkonchelate aufzeigen, wobei die möglichen Anwendung nicht auf die Beispiele beschränkt sein sollen.

## Beispiel 1

Herstellung von n-Propoxy-bis-2-(methoxyethoxy)-ethoxy-2,4-pentandionato-zirkon

In den 1 l-Kolben eines Labor-Vacuumrotationsverdampfers werden 409,5 g handelsübliches Propylzirkonat (0,9 Mol Tetra-n-propoxy-zirkon als 72 %ige Lösung in Propanol-1) eingewogen und zunächst mit 216 g Methyldiglykol (1,8 Mol, 2-(2-Methoxyethoxy) ethanol) versetzt. Anschließend werden in diese Mischung 90 g Acetylaceton (0,9 Mol, Pentandion-2,4) portionsweise eingerührt und das Reaktionsprodukt unter Rühren 30 Minuten auf 80 °C erwärmt. Nach Abkühlung auf 45 bis 50 °C wird aus dem so erhaltenen Reaktionsgemisch sowohl das im Ausgangsmaterial enthaltene wie auch das durch die Reaktionen des Propylzirkonats mit dem Methyldiglykol und dem Acetylaceton entstandene Propanol-1 durch Destillation unter vermindertem Druck (ca. 20-25 mbar) entfernt. (Destillat-Ausbeute: 272,5 g ≙ 98,5 % d. Theorie). Das so erhaltene Produkt ist eine gelbe, viskose Flüssigkeit mit folgenden Kennzahlen:

| | |
|---|---|
| Brechungszahl $n_D^{20}$ = | 1,5095-1,5110 |
| Viskosität (20 °C) = | 6 000-12 000 mPa · s |
| Zirkondioxid-Gehalt = | 25,0-25,6 % |

Löslichkeit:

a) in Wasser: als 2,5 %ige Lösung klar löslich

b) in organischen Lösungsmitteln: als 10 %ige Lösungen in Isopropanol, Methylethylketon, Ethylacetat, Methylenchlorid, Toluol und Heptan klar löslich.

Die genannten Lösungen sind mindestens 6 Wochen bei Raumtemperatur stabil.

Beispiel 2

Herstellung von Di-n-propoxy-2-(methoxyethoxy)-ethoxy-2,4-pentandionato-zirkon

In den 1 l-Kolben eines Labor-Vacuumrotationsverdampfers werden 455 g handelsübliches Propylzirkonat (1 Mol Tetra-n-propoxy-zirkon als 72 %ige Lösung in Propanol-1) eingewogen und unter Rühren nacheinander mit 120 g Methyldiglykol (1 Mol, 2-(2-Methoxyethoxy)-ethanol) und mit 100 g Acetylaceton (1 Mol, Pentandion-2,4) versetzt. Die so erhaltene Reaktionsmischung wird durch Destillation unter vermindertem Druck von dem durch die Reaktionen entstandenen und dem im Ausgangsmaterial enthaltenen Propanol-1 befreit (Destillat-Ausbeute : 245,7 g ≙ 99,3 % d. Theorie). Es ergibt sich eine gelbe, viskose Flüssigkeit mit folgenden Kennzahlen :

| | |
|---|---|
| Brechungszahl $n_D^{20}$ = | 1,5175-1,5195 |
| Viskosität (20 °C) = | 1 100-1 600 mPa · s |
| Zirkondioxid-Gehalt = | 28,5-29,1 % |

Löslichkeit :

a) in Wasser : als 2,5 %ige Lösung klar löslich

b) in organischen Lösungsmitteln : als 10 %ige Lösungen in Isopropanol, Methylethylketon, Ethylacetat, Methylenchlorid, Toluol und Heptan klar löslich.

Die genannten Lösungen sind mindestens 6 Wochen bei Raumtemperatur stabil.

Beispiel 3

Herstellung von Butoxy-bis-2-(2-methoxyethoxy) ethoxy-2,4-pentandionato-zirkon

In den 1 l-Kolben eines Labor-Vacuumrotationsverdampfers werden 409,5 g handelsübliches Butylzirkonat (0,9 Mol Tetra-n-butoxy-zirkon als 84 %ige Lösung in Butanol-1) eingewogen und nacheinander mit 216 g Methyldiglykol (1,8 Mol, 2-(2-Methoxyethoxy)-ethanol) und mit 90 g Acetylaceton (0,9 Mol, Pentandion-2,4) versetzt. Diese Mischung wird auf 50 °C erwärmt und anschließend im Vacuum vom darin enthaltenen Butanol-1 befreit, das aus der Reaktion des Butylzirkonats mit Methyldiglykol und Acetylaceton sowie aus dem Ausgangs-Butylzirkonat stammt. Die Abtrennung des Butanol-1 findet bei 20 bis 25 mbar und einer maximalen Wasserbadtemperatur von 80 °C statt. (Destillat-Ausbeute : 264,0 g ≙ 99,5 % d. Theorie).

Das so erhaltene Produkt ist eine orange, viskose Flüssigkeit mit folgenden Kennzahlen :

| | |
|---|---|
| Brechungszahl $n_D^{20}$ = | 1,5080-1,5110 |
| Viskosität (20 °C) = | 600-850 mPa · s |
| Zirkondioxid-Gehalt = | 24,5-25,2 % |

Löslichkeit :

a) in Wasser : als 2,5 %ige Lösung klar löslich

b) in organischen Lösungsmitteln : als 10 %ige Lösung in Isopropanol, Methylethylketon, Ethylacetat, Methylenchlorid, Toluol und Heptan klar löslich.

Die genannten Lösungen sind mindestens 6 Wochen bei Raumtemperatur stabil.

Beispiel 4

Herstellung von n-Propoxy-bis-(2-ethoxyethoxy)-2,4-pentandionato-zirkon

In den 1 l-Kolben eines Labor-Vacuumrotationsverdampfers werden 455 g handelsübliches Propylzirkonat (1 Mol Tetra-n-propoxy-zirkon als 72 %ige Lösung in n-Propanol) eingewogen und mit 180 g Ethylglykol (2-Ethoxyethanol) versetzt. Diese Mischung wird auf 40 °C erwärmt und im Vacuum (20 bis 25 mbar) von ca. 75 % des darin enthaltenen n-Propanols befreit. Zu diesem Produkt werden anschließend 100 g Acetylaceton (1 Mol, Pentandion-2,4) gegeben und auf 50 °C erwärmt. Im Vacuum werden dann dieser Reaktionsmischung alle in Form von n-Propanol vorliegenden flüchtigen Bestandteile abgezogen, wobei die Temperatur bis auf 80 °C gesteigert wird. Destillat-Ausbeute (Stufe 1 + 2) : 304,0 g = 98,9 % d. Theorie.

Das so erhaltene Produkte ist eine orangerote, viskose Flüssigkeit mit folgenden Kennzahlen :

| | |
|---|---|
| Brechungszahl $n_D^{20}$ = | 1,5085-1,5105 |

| | |
|---|---|
| Viskosität (20 °C) = | 7 500-10 000 mPa · s |
| Zirkondioxid-Gehalt = | 28,6-29,3 % |

Löslichkeit:

a) in Wasser: als 2,5 %ige Lösung klar löslich
b) in organischen Lösungsmitteln: als 10 %ige Lösung in Isopropanol, Methylethylketon, Ethylacetat, Methylenchlorid, Toluol, und Heptan klar löslich.

Beispiel 5

Verwendung des Produktes nach Beispiel 2 als Veresterungskatalysator bei der Herstellung eines Polyesters aus Butandiol-1,4 und Adipinsäure.

In eine Veresterungsapparatur mit Rührer, Thermometer, Wasserabscheider und Rückflußkühler werden 146 g (1 Mol) Adipinsäure und 99,4 g (1,1 Mol) Butandiol-1,4 eingewogen und soweit erhitzt, daß sich die Komponenten zu einer homogenen Mischung auflösen. Bei dieser Temperatur (etwa 150 °C) werden 0,15 g des Produktes nach Beispiel 2 zugegeben und weiter erhitzt, bis bei etwa 170 bis 180 °C die Wasserabspaltung einsetzt. Nach 25 Minuten sind 70 % der theoretischen Wassermenge abgespalten, und es wird zur Vervollständigung der Veresterung unter Vacuum (20 bis 25 mbar) bei 200 °C 2 Stunden nachbehandelt. Der so erhaltene Polyester weist eine Säurezahl von 5,6 auf und ist im geschmolzenen Zustand klar. Bei einem zum Vergleich mit 0,15 g Isopropyltitanat als Katalysator hergestellten Polyester dauert es 75 Minuten, bis 70 % der theoretischen Wassermenge abgespalten sind. Dieser Vergleichspolyester wird nach ebenfalls 2 Stunden Vacuumnachkondensation mit einer Säurezahl von 5,0 erhalten, ist aber im geschmolzenen Zustand deutlich trübe.

Beispiel 6

Verwendung des Produktes nach Beispiel 2 als Bestandteil eines haftvermittelnden Primers auf wässriger Basis Streifen einer handelsüblichen, nicht vorbehandelten Polyethylenterephthalat-Folie werden 30 Sekunden in eine 1 %ige wässrige Lösung des in Beispiel 2 beschriebenen Produkts getaucht, anschließend 30 Minuten an der Luft und 60 Minuten im Umluftschrank bei 60 °C getrocknet und dann mit einem ca. 20 % Festkörper enthaltenden, wenig mit Titandioxid pigmentierten Prüflack in einer Naßschichtdicke von ca. 0,04 mm überzogen. Dieser Lack wird nach Lufttrocknung über Nacht und Nachbehandeln im Umluftschrank (3 Stunden, 60 °C) mittels Tesafilm-Abrißtest auf seine Haftung auf der Folie geprüft. Es zeigte sich, daß Lacke mit Nitrocellulose (sowohl ester- wie auch alkohollösliche Typen), Celluloseacetopropionat oder Celluloseacetobutyrat als Bindemittel auf so vorbehandelten Folien vom Tesafilm nicht abgerissen werden, wogegen diese Lacke, auf unvorbehandelte Folien aufgebracht, bei dieser Prüfung vollständig abgezogen werden.

## Patentansprüche

1. Zirkonsäureester der Formel

$$Zr(OR)_n(OR')_o\,(C_5H_7O_2)_p$$

in der
R = Alkylreste mit 1 bis 8 C-Atomen,
R' = $-[CH_2-CHR''-O]_q-R$,
R'' = H oder Methylgruppe bedeuten,
der Rest $C_5H_7O_2$ für den Acetylacetonylrest steht
und
n = 0-2,
o = 1-3,
p = 1-2 sein kann mit der Bedingung, daß n + o + p = 4 ist
und
q = Werte von 1-8 annehmen kann.

2. Verfahren zur Herstellung von Zirkonsäureestern gemäß Anspruch 1, dadurch gekennzeichnet, daß man Zirkonsäureester der Formel $Zr(OR)_4$, in der R die oben genannte Bedeutung hat, mit 1 bis 3 Mol Glycolethern oder Glycoletherestern und mit bis zu 2 Mol Acetylaceton umsetzt und den freigesetzten Alkohol auf an sich bekannte Weise abdestilliert.

3. Verwendung von Zirkonsäureverbindungen gemäß Anspruch 1 in wasserhaltigen oder wässrigen Systemen als Katalysatoren, Vernetzer für Polyhydroxyverbindungen oder Additive in Wasserlacksystemen.

## Claims

1. Zirconic acid esters of the formula

$$Zr(OR)_n(OR')_o\ (C_5H_7O_2)_p$$

in which
R denotes alkyl residues with 1 to 8 C-atoms
R' denotes $—[CH_2—CHR''—O]_q—R$,
R'' = H or a methyl group,
the residue $C_5H_7O_2$ stands for the acetylacetonyl residue
and
n can be 0-2,
o can be 1-3,
p can be 1-2, with the requirement that n + o + p = 4
and
q can adopt a value of 1-8.

2. Process for the production of zirconic acid esters according to claim 1, characterised in that zirconic acid esters of the formula $ZR(OR)_4$ are reacted with 1 to 3 mol of glycol ethers or glycolether esters and with up to 2 mol of acetylacetone and the liberated alcohol is distilled off in a manner known per se.

3. Use of zirconic acid compounds according to claim 1 in water-containing or aqueous systems as catalysts, cross-linkers for polyhydroxy compounds or additives in aqueous lacquer systems.

## Revendications

1. Ester de l'acide zirconique, de formule :

$$Zr(OR)_n(OR')_o\ (C_5H_7O_2)_p$$

dans laquelle :
R représente des restes alkyles ayant 1 à 8 atomes de carbone,
R' représente $—[CH_2—CHR''—O]_q—R$,
R'' représente H ou un groupe méthyle,
le reste $C_5H_7O_2$ représente le reste acétylacétonyle,
et
n peut valoir 0 à 2,
o peut valoir 1 à 3,
p peut valoir 1 à 2, à la condition que la somme (n + o + p) vale 4,
et
q peut prendre des valeurs allant de 1 à 8.

2. Procédé pour préparer des esters de l'acide zirconique selon la revendication 1, caractérisé en ce qu'on fait réagir un ester d'acide zirconique de formule $Zr(OR)_4$, dans laquelle R a le sens précité, avec 1 à 3 moles d'éthers de glycols ou d'esters éthers de glycols avec jusqu'à 2 moles d'acétylacétone, et l'on chasse par distillation, de façon connue en soi, l'alcool libéré.

3. Utilisation des composés de l'acide zirconique selon la revendication 1 dans des systèmes contenant de l'eau ou dans des système aqueux, à titre de catalyseurs, d'agents de réticulation de composés polyhydroxylés ou d'additifs dans des systèmes de laques et vernis à l'eau.